(19) Europäisches Patentamt
European Patent Office
Office européen des brevets

(11) Veröffentlichungsnummer : **0 410 250 B1**

(12) **EUROPÄISCHE PATENTSCHRIFT**

(45) Veröffentlichungstag der Patentschrift :
**05.01.94 Patentblatt 94/01**

(51) Int. Cl.$^5$ : **C07C 381/12,** C08F 2/50, G03F 7/004

(21) Anmeldenummer : **90113552.5**

(22) Anmeldetag : **16.07.90**

# (54) Neue Sulfoniumsalze und deren Verwendung.

(30) Priorität : **22.07.89 DE 3924299**

(43) Veröffentlichungstag der Anmeldung :
**30.01.91 Patentblatt 91/05**

(45) Bekanntmachung des Hinweises auf die Patenterteilung :
**05.01.94 Patentblatt 94/01**

(84) Benannte Vertragsstaaten :
**BE CH DE FR GB LI NL**

(56) Entgegenhaltungen :
**EP-A- 0 297 442**

(56) Entgegenhaltungen :
**DE-A- 3 604 581**
**DE-A- 3 721 741**
**US-A- 2 833 827**
**US-A- 4 451 409**

(73) Patentinhaber : **BASF Aktiengesellschaft**
**Carl-Bosch-Strasse 38**
**D-67063 Ludwigshafen (DE)**

(72) Erfinder : **Schwalm, Reinhold, Dr.**
**Am Huettenwingert 53**
**D-6706 Wachenheim (DE)**
Erfinder : **Boettcher, Andreas, Dr.**
**Konrad-Adenauer-Ring 38**
**D-6907 Nussloch (DE)**

EP 0 410 250 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Sulfoniumsalze, die zusätzlich zur Sulfoniumgruppierung bestimmte funktionale Gruppierungen enthalten, sowie strahlungsempfindliche Gemische, die diese Sulfoniumsalze enthalten. Diese Sulfoniumsalze eignen sich als Photoinitiatoren zur photoinitiierten kationischen Polymerisation sowie zur Herstellung von Reliefbildern und Reliefmustern.

Sulfoniumsalze sind seit langem in der Literatur bekannt, vgl. z.B. H.M. Pitt, US-A-2, 807, 648 (1957); W. Hahn und R. Stroh, US-A-2, 833, 827 (1958); G.H. Wiegand and W.E. McEwen, J.Org.Chem., 33, 2671 (1968).

Als Photoinitiatoren kommen fast ausschließlich Sulfoniumsalze mit komplexen, nichtnucleophilen Gegenionen in Frage, wie die von Crivello entwickelten Photoinitiatoren für die kationische Polymerisation (vgl. z.B. US-A-4, 058, 400 und US-A-4, 058, 401). Einen Überblick über die Verwendung von Oniumsalzen bei der kationischen Polymerisation gibt Crivello in "Cationic Polymerisation - Iodonium and Sulfonium Salt Photoinitiators, Advances in Polym.Sci. 62, 1-48 (1984)".

Die Verwendung von Oniumsalzen in Photoresistmaterialien ist z.8. in "Possibilities for Photoimaging Using Onium Salts, Crivello in Corporate Research and Development, General Electric, Schenectady, New York (1983)", sowie von Ito and Willson in org.Ctgs. and App.Polym.Sci.Proc. 48, 60 (1983) und US-A-4,491,628 beschrieben.

Die bisher beschriebenen Sulfoniumsalze sind sehr effektive Initiatoren für die Polymerisation bzw. effektive Säurespender in Photoresistmaterialien; keines der oben genannten Sulfoniumsalze enthält jedoch säurelabile Gruppen, die durch Einwirkung von Strahlung abgespalten werden können, und das Löslichkeitsverhalten der Verbindungen drastisch ändern.

In der DE-A 37 21 740 sind Sulfoniumsalze der allgemeinen Formel

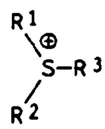

$X^{\ominus}$

beschrieben, worin $R^1$, $R^2$ und $R^3$ untereinander gleich oder veschieden sind und für aliphatische und/oder aromatische Reste, die gegebenenfalls Heteroatome enthalten, stehen oder zwei der Reste $R^1$ bis $R^3$ miteinander zu einem Ring verknüpft sind, mit der Maßgabe, daß mindestens einer der Reste $R^1$ bis $R^3$ mindestens eine durch Säure spaltbare Gruppierung enthält, wobei einer der Reste $R^1$ bis $R^3$ mit einem oder mehreren weiteren Sulfoniumsalzresten, gegebenenfalls über durch Säure spaltbare Gruppierungen, verknüpft sein kann, und $X^{\ominus}$ ein nichtnucleophiles Gegenion bedeutet. Als bevorzugte, durch Säure spaltbare Gruppierungen sind hier tert.-Butoxicarbonyl und Trialkylsilylgruppen genannt.

Ether und Acetale/Ketale als Schutzgruppen für Phenole sind schon lange bekannt (vgl. z.B. Protective Groups in Organic Synthesis, Th.W. Greene, Ed., John Wiley & Sons, New York (1980), Kapitel 4: "Protection of Phenols and Catechols").

Aufgabe der vorliegenden Erfindung war es, aus den für Phenole möglichen Schutzgruppen solche aufzuzeigen, die für Sulfoniumsalze geeignet sind, um so Photoinitiatoren zur Verfügung zu stellen, die eine hohe Sensitivität aufweisen, möglichst in einem breiten Bereich des Spektrums der elektromagnetischen Wellen effektiv sind und ihr Löslichkeitsverhalten durch die Belichtung drastisch ändern.

Diese Aufgabe läßt sich sehr vorteilhaft durch die speziellen Sulfoniumsalze gemäß der vorliegenden Erfindung lösen.

Gegenstand der vorliegenden Erfindung sind Sulfoniumsalze der allgemeinen Formel (I)

$$R_{3-x}-\overset{\oplus}{S}-(R'-O-R'')_{x} \quad A^{\ominus} \qquad (I)$$

worin

$A^{\ominus}$ = nichtnucleophiles Gegenion,

x = 1, 2 oder 3,

R = Alkyl, Cycloalkyl, Aryl, substituiertes Aryl oder für den Fall x = 1 ein divalenter cyclischer Rest mit $S^{\oplus}$ als Ringglied,

R' = Arylen, substituiertes Arylen,
R'' =

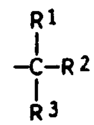

worin $R^1$, $R^2$ und $R^3$ untereinander gleich oder verschieden sind und für Alkyl mit 1 bis 18 Kohlenstoffatomen, für ein- oder mehrfach halogensubstituiertes Alkyl mit der Maßgabe, daß mindestens einer der Reste $R^1$ bis $R^3$ am $\alpha$-Kohlenstoff ein Wasserstoffatom enthält,
oder
$R^1$ und $R^2$ untereinander gleich oder verschieden sind und für Wasserstoff oder Alkyl stehen, und $R^3$ für Phenyl oder substituiertes Phenyl steht,
oder
$R^1$ und $R^2$ untereinander gleich oder verschieden sind und für Wasserstoff oder Alkyl stehen, und $R^3$ für Alkenyl oder Cycloalkenyl steht,
oder
$R^1$ für Wasserstoff steht, und $R^2$ und $R^3$ zu einem, vorzugsweise fünf- oder sechsgliedrigen, ethylenisch ungesättigten Ring geschlossen sind,
oder
$R^1$ und $R^2$ untereinander gleich oder verschieden sind und für Wasserstoff, Alkyl, Cycloalkyl oder Aryl stehen, und $R^3$ für Alkoxy steht oder $R^3$ mit $R^2$ über die Gruppe $-O-(CH_2)_n-$ mit n = 3 bis 6 zu einem Ring geschlossen ist,
oder
$R^1$ für Wasserstoff oder Alkyl steht, $R^2$ und $R^3$ untereinander gleich oder verschieden sind und für Alkoxy, Aryloxy oder substituiertes Aryloxy stehen.

Bevorzugt sind solche erfindungsgemäßen Sulfoniumsalze, in welchen in der allgemeinen Formel (I) R'' für einen tert.-Alkylrest, einen Benzylrest, einen Allylrest, einen Ketalrest oder einen Acetalrest steht.

Gegenstand der vorliegenden Erfindung sind außerdem strahlungsempfindliche Gemische, die ein erfindungegemäßes Sulfoniumsalz enthalten. Gegenstand der vorliegenden Erfindung ist ferner ein Verfahren zur photoinitiierten kationischen Polymerisation, wobei als Photoinitiator ein erfindungsgemäßes Sulfoniumsalz eingesetzt wird sowie Verfahren zur Herstellung von Reliefmustern oder Reliefbildern, wobei jeweils mindestens ein erfindungsgemäßes Sulfoniumsalze als Photoinitiator und Löslichkeitsinhibitor eingesetzt wird.

In der allgemeinen Formel (I)

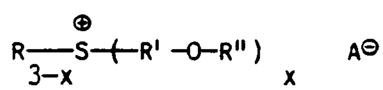

(I)

für die erfindungsgemäßen Sulfoniumsalze bedeuten:

$A^{\ominus}$ = nichtnucleophiles Gegenion, vorzugsweise ein komplexes, nichtnucleophiles Metallhalogenid, wie z.B. $BF_4^{\ominus}$, $AsF_6^{\ominus}$, $SbF_6^{\ominus}$ und/oder $PF_6^{\ominus}$ sowie Trifluormethansulfonat,
x = 1, 2 oder 3,
R = Alkyl, beispielsweise mit 1 bis 6, vorzugsweise 1 bis 4 Kohlenstoffatomen, wie Methyl, Ethyl, Butyl, Cycloalkyl, beispielsweise mit 5 bis 7 Kohlenstoffatomen, wie z.B. Cyclohexyl, Aryl, beispielsweise Phenyl oder Naphthyl, substituiertes Aryl wie z.B. t-Butyl-phenyl oder für den Fall x = 1 ein divalenter cyclischer Rest mit $S^{\oplus}$ als Ringglied, z.B. ein Tetrahydrothiophenring,
R' = Arylen, wie z.B. 1,4-Phenylen, substituiertes Arylen, wie z.B. 2-Methoxy-1, 4-phenylen,
R'' =

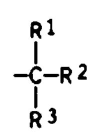

worin $R^1$, $R^2$ und $R^3$ untereinander gleich oder verschieden sind und für Alkyl mit 1 bis 18, vorzugsweise 1 bis 6 Kohlenstoffatomen, für ein- oder mehrfach halogensubstituiertes Alkyl, wie z.B. Trifluormethyl, mit der Maßgabe, daß mindestens einer der Reste $R^1$ bis $R^3$ am α-Kohlenstoff ein Wasserstoffatom enthält,

oder

$R^1$ und $R^2$ untereinander gleich oder verschieden sein können und für Wasserstoff oder Alkyl, z.B. mit 1 bis 6 Kohlenstoffatomen stehen, und $R^3$ für Phenyl oder substituiertes Phenyl steht,

oder

$R^1$ und $R^2$ untereinander gleich oder verschieden sind und für Wasserstoff oder Alkyl, z.B. mit 1 bis 6 Kohlenstoffatomen stehen, und $R^3$ für Alkenyl, z.B. mit 3 bis 6 Kohlenstoffatomen, wie z.B. Allyl, Methallyl, oder Cycloalkenyl, wie z.B. Cyclohexenyl steht,

oder

$R^1$ für Wasserstoff steht, und $R^2$ und $R^3$ zu einem, vorzugsweise fünf- oder sechsgliedrigen, ethylenisch ungesättigten Ring geschlossen sind, z.B. Cyclohexenyl

oder

$R^1$ und $R^2$ untereinander gleich oder verschieden sind und für Wasserstoff, Alkyl, z.B. mit 1 bis 6 Kohlenstoffatomen, Cycloalkyl, wie z.B. Cyclohexyl oder Aryl, wie z.B. Phenyl oder Naphthyl stehen, und $R^3$ für Alkoxy, z.B. mit 1 bis 6 Kohlenstoffatomen steht oder $R^3$ mit $R^2$ über die Gruppe $-O-(CH_2)_{\overline{n}}$ mit n = 3 bis 6, vorzugsweise n = 4, zu einem Ring geschlossen ist,

oder

$R^1$ für Wasserstoff oder Alkyl, z.B. mit 1 bis 6 Kohlenstoffatomen steht, $R^2$ und $R^3$ untereinander gleich oder verschieden sind und für Alkoxy, z.B. mit 1 bis 6 Kohlenstoffatomen, Aryloxy wie z.B. Phenoxy oder substituiertes Aryloxy, wie z.B. Alkyl-phenoxy stehen.

Als Schutzgruppen für die phenolische Funktion an Sulfoniumsalzen kommen erfindungsgemäß bevorzugt solche Gruppen in Betracht, welche sich leicht mit Hilfe von Säuren abspalten lassen, beispielsweise tert.-Alkylgruppen wie t-Butyl oder t-Amyl, Allylgruppen wie Allyl, Cyclohexenyl oder Alkylcyclohexenyl und Benzylgruppen wie Benzyl, α-Methylbenzyl oder α,α-Dimethylbenzyl sowie Acetale, wie Tetrahydropyranylgruppen.

Zur Synthese derartiger erfindungsgemäßer Sulfoniumsalze ist im einzelnen folgendes auszuführen.

Bekannt sind drei allgemeine Reaktionstypen zur Alkylierung von Phenolen:

a) Reaktion des freien Phenols mit Diazoalkanen

b) Reaktion von Alkylhalogeniden mit Phenol in Gegenwart einer Base und

c) Reaktion eines Phenols mit einem Olefin unter Säurekatalyse.

Bei der Umsetzung der entsprechenden phenolischen Sulfoniumsalze hat sich besonders Methode b), die Umsetzung der entsprechenden phenolischen Sulfoniumsalze mit Alkylhalogeniden bewährt. Eine weitere Synthesemöglichkeit für die Herstellung der entsprechenden Sulfoniumsalze ist die Kondensation von Alkylphenylethern mit Thionylchlorid zu den entsprechenden Sulfoniumsalzen (vgl. z.B. C.A., 64, 19466 (1966) und D. Libermann, Compt. R. 197, 1425 (1933)).

Die Synthese der als Ausgangsmaterialien verwendeten Hydroxiphenylsulfoniumsalze ist bekannt (vgl. J.Polym.Sci., Chem.Ed., 18, 1021 (1980); US-A-2 833 827 (1958) und DE-A 37 21 741).

Diese Sulfoniumsalze kann man analog den Synthesevorschriften für die Umsetzung von Hydroxyphenylverbindungen mit Allyl- bzw. Benzylhalogeniden, z.B. in J.Amer. Chem. Soc., 89, 6745 (1967); J.Amer.Chem.Soc., 73, 5765 (1951); Chem.Ber. 88, 91 (1955) oder Tetrahedron Letters, 343 (1970) mit den entsprechenden Allyl-bzw. Benzylhalogeniden in Gegenwart einer Base unter homogenen oder heterogenen (Phasentransferkatalyse) Reaktionsbedingungen zu den entsprechend funktionalisierten Sulfoniumsalzen umsetzen.

Die Synthese der Sulfoniumsalze, die Acetalgruppierungen enthalten, kann analog den in Helv.Chim.Acta 46, 415 (1963) und Synthesis, S. 153ff (1974) sowie organic Chemistry, S. 1-133, Academic Press, New York (1970) beschriebenen Methoden durchgeführt werden.

Die Einführung von Orthocarbonsäureestergruppierungen in die Salze kann beispielsweise durch Umsetzung eines Hydroxyphenylsulfoniumsalzes mit Diphenoxychlormethan in Methylenchlorid, Tetrahydrofuran oder Ethylacetat in Gegenwart einer Base, z.B. Triethylamin, durchgeführt werden (vgl. auch beispielsweise US-A-4 101 323, Spalte 16, Zeilen 40ff).

Die Verwendung der Sulfoniumsalze als Photoinitiatoren für die Abspaltung von säurelabilen Seitengruppen in Photoresistmaterialien läßt sich zeigen, indem eine Photoresistlösung aus Poly-(t-butyl-methacrylat) und 20 Gew.% bezogen auf das Polymere, eines erfindungsgemäßen Sulfoniumsalzes, auf einen Siliziumwafer aufgeschleudert, bildmäßig bestrahlt, ausgeheizt und entwickelt wird. Die belichteten Bereiche lassen sich mit einem alkalischen Entwickler vollständig abtragen, während in den unbelichteten Bereichen kein Abtrag stattfindet. Bei Einsatz der üblicherweise verwendeten Triarylsulfoniumsalze bleibt eine dünne

Restschicht zurück bzw. ist zum sauberen Entwickeln ein Cosolvens zum Lösen der Sulfoniumsalze notwendig.

Die erfindungsgemäßen Sulfoniumsalze eignen sich als Photoinitiatoren für die photoinitiierte kationische Polymerisation von z.B. Styrol, Alkylvinylethern, cyclischen Ethern und Epoxiden.

Die erfindungsgemäßen Sulfoniumsalze eignen sich auch sehr vorteilhaft als Photoinitiatoren und Löslichkeitsinhibitoren bei der Herstellung von Photoresistmaterialien, wobei als polymere Bindemittel bevorzugt phenolische Harze, wie Novolake oder Poly-p-hydroxystyrol in Frage kommen.

Die in den Beispielen genannten Teile und Prozente sind, soweit nicht anders angegeben, Gewichtsteile bzw. Gewichtsprozente.

Beispiel 1

Synthese von Tris(-Methylbenzyl-oxiphenyl)sulfoniumhexafluoroarsenat

5 Teile Tris-(4-hydroxiphenyl)-sulfoniumhexafluoroarsenat werden in 100 Teilen trockenem Tetrahydrofuran unter $N_2$-Durchleitung gelöst. Dann gibt man 3,36 Teile Kalium-t-butylat zu und rührt anschließend noch 10 Minuten nach. Dazu tropft man 5,55 Teile $\alpha$-Methylbenzylbromid und rührt über Nacht nach. Die Reaktionslösung wird in 150 Teile Eiswasser gegeben und mit Ethylacetat mehrmals extrahiert. Die kombinierten Ethylacetatfraktionen werden über ein Molekularsieb getrocknet, das Lösungsmittel wird abgezogen. Nach dem Umkristallisieren erhält man 4,7 Teile (78 %) reines Produkt. IR- und H-NMR-spektroskopische Analyse stimmen mit der erwarteten Struktur überein.

Beispiele 2 bis 4

Es wird wie in Beispiel 1 verfahren, jedoch werden anstelle des $\alpha$-Methylbenzylbromids folgende Halogenide eingesetzt:

| Nr. | Halogenid | Ausbeute | Charakterisierung |
|---|---|---|---|
| 2 | Benzylbromid | 68 % | IR, NMR |
| 3 | Cyclohexenylbromid | 82 % | IR, NMR |
| 4 | Allylbromid | 53 % | IR |

Beispiel 5

Synthese von Tris-(t-Butyloxiphenyl)sulfoniumhexafluorophosphat

2 Mol Thionylchlorid und 3 Mol t-Butyl-phenyl-ether werden in einem Reaktionskolben, der mit Eiswasser gekühlt wird, zusammengegeben. Dann wird unter Rühren portionsweise pulverisiertes Aluminiumchlorid zugegeben, bis keine Gasentwicklung mehr zu beobachten ist. Der Reaktionsansatz wird über Nacht stehengelassen und anschließend in Eiswasser gegeben. Das Reaktionsprodukt scheidet sich als viskoses Öl ab. Die ölige Phase wird dann mit Diethylether gewaschen, um nichtumgesetztes Ausgangsprodukt zu entfernen. Das erhaltene Rohprodukt wird in Methanol gelöst und mit der stöchiometrischen Menge Silberhexafluorophosphat, gelöst in Methanol, versetzt. Es wird von ausgefallenem Silberchlorid abfiltriert und das Lösungsmittel abgezogen. Die Gesamtausbeute an Produkt beträgt 18 %. IR und H-NMR spektroskopische Untersuchungen stimmen mit der erwarteten Struktur überein.

Beispiel 6

Es wird wie in Beispiel 5 verfahren, jedoch wird anstelle von Silberhexafluorophosphat mit Silberhexafluoroarsenat versetzt. Ausbeute an charakterisiertem Produkt: 21 %.

Beispiel 7

Synthese von Tris-(tetrahydropyranyl-oxyphenyl) sulfoniumhexafluoroarsenat

4 Teile Tris-(hydroxyphenyl)sulfoniumchlorid (hergestellt wie beschrieben in Carre et Libermann, Comptes

rendus, 196, 275 (1933)) werden mit 25 Teilen Dihydropyran und einer katalytischen Menge Salzsäure versetzt und 72 Stunden bei Raumtemperatur gerührt. Der Feststoff wird abgesaugt, mehrmals mit Wasser gewaschen und getrocknet. Nach chromatographischer Trennung resultieren 2,1 Teile Produkt der erwarteten Struktur. Das tetrahydropuranyl funktionalisierte Sulfoniumchlorid wird in Methanol/Dimethylformamid gelöst und die equimolare Menge Silberarsenat, gelöst in Methanol, zugetropft. Es wird von ausgefallenem Silberchlorid abfiltriert und die Lösung im Rotationsverdampfer eingedampft. Die Ausbeute an Sulfoniumhexafluoroarsenat beträgt 1,8 Teile.

Beispiel 8

Synthese von Tris-(Diphenoxymethyl-oxyphenyl)sulfoniumhexafluoroarsenat

20 Teile Tris-(hydroxyphenyl)sulfoniumhexafluoroarsenat werden in 100 Teilen Tetrahydrofuran gelöst. Dazu gibt man 12,2 Teile Triethylamin und tropft eine Lösung von 30 Teilen Diphenoxychlormethan langsam zu und rührt 24 Stunden bei Raumtemperatur. Die Reaktionsmischung wird anschließend mit Wasser gewaschen, die organische Lösung über Molekularsieb getrocknet und dann im Rotationsverdampfer eingedampft. Nach chromatographischer Reinigung erhält man 14,8 Teile des erwarteten Produkts. IR und NMR stimmen mit der erwarteten Struktur überein.

Beispiel 9

Eine Photoresistlösung wird aus 1 Teil des wie in Beispiel 6 synthetisierten Sulfoniumsalzes, 3 Teilen eines Poly-(p-hydroxystyrols) mit dem Molekulargewicht 31,000 g/mol (Lichtstreuung) und 7 Teilen Methylpropylenglykol-acetat hergestellt. Die Lösung wird durch ein Filter mit einem Porendurchmesser von 0,2 µm filtriert und mit einer Schichtdicke von ca. 1 µm auf einen Siliziumwafer aufgeschleudert. Nach dem Ausheizen bei 90°C (1 Minute) wird durch eine in Kontakt mit der Waferoberfläche gebrachte Testmaske mit Excimerlaserlicht der Wellenlänge 248 nm während 10 Sekunden belichtet. Anschließend heizt man eine weitere Minute bei 90°C aus und entwickelt 60 Sekunden mit einem alkalischen Entwickler vom pH 13,0. An den belichteten Stellen ist der Resist vollständig abgetragen, während die unbelichteten Bereiche nur einen Abtrag von 3 % erleiden. Es entstehen Resistmuster guter Qualität.

## Patentansprüche

1. Sulfoniumsalze der allgemeinen Formel (I)

$$R_{3-x}\overset{\oplus}{S}\left(R'-O-R''\right)_x \quad A^{\ominus} \qquad (I)$$

worin

| | |
|---|---|
| $A^{\ominus}$ = | nichtnucleophiles Gegenion, |
| x = | 1, 2 oder 3, |
| R = | Alkyl, Cycloalkyl, Aryl, substituiertes Aryl oder für den Fall x = 1 ein divalenter cyclischer Rest mit $S^{\oplus}$ als Ringglied, |
| R' = | Arylen, substituiertes Arylen, |
| R" = | |

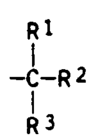

worin $R^1$, $R^2$ und $R^3$ untereinander gleich oder verschieden sind und für Alkyl mit 1 bis 18 Kohlenstoffatomen, für ein- oder mehrfach halogensubstituiertes Alkyl mit der Maßgabe, daß mindestens einer der Reste $R^1$ bis $R^3$ am α-Kohlenstoff ein Wasserstoffatom enthält, oder

$R^1$ und $R^2$ untereinander gleich oder verschieden sind und für Wasserstoff oder Alkyl stehen, und $R^3$ für Phenyl oder substituiertes Phenyl steht,
oder
$R^1$ und $R^2$ untereinander gleich oder verschieden sind und für Wasserstoff oder Alkyl stehen, und $R^3$ für Alkenyl oder Cycloalkenyl steht,
oder
$R^1$ für Wasserstoff steht, und $R^2$ und $R^3$ zu einem, vorzugsweise fünf- oder sechsgliedrigen, ethylenisch ungesättigten Ring geschlossen sind,
oder
$R^1$ und $R^2$ untereinander gleich oder verschieden sind und für Wasserstoff, Alkyl, Cycloalkyl oder Aryl stehen, und $R^3$ für Alkoxy steht oder $R^3$ mit $R^2$ über die Gruppe $-O-(CH_2)_n-$ mit n = 3 bis 6 zu einem Ring geschlossen ist,
oder
$R^1$ für Wasserstoff oder Alkyl steht, $R^2$ und $R^3$ untereinander gleich oder verschieden sind und für Alkoxy, Aryloxy oder substituiertes Aryloxy stehen.

2. Sulfoniumsalze nach Anspruch 1, dadurch gekennzeichnet, daß in der allgemeinen Formel (I) R" für einen tert.-Alkyl- oder Benzylrest steht.

3. Sulfoniumsalze nach Anspruch 1, dadurch gekennzeichnet, daß in der allgemeinen Formel (I) R" für einen Allyl-, Ketal- oder Acetalrest steht.

4. Strahlungsempfindliches Gemisch, dadurch gekennzeichnet, daß es ein Sulfoniumsalz gemäß einem der vorhergehenden Ansprüche enthält.

5. Verfahren zur photoinitiierten kationischen Polymerisation, dadurch gekennzeichnet, daß als Photoinitiator ein Sulfoniumsalz gemäß einem der Ansprüche 1 bis 3 eingesetzt wird.

6. Verfahren zur Herstellung von Reliefmustern oder Reliefbildern, dadurch gekennzeichnet, daß mindestens ein Sulfoniumsalz gemäß einem der Ansprüche 1 bis 3 als Photoinitiator und Löslichkeitsinhibitor eingesetzt wird.

## Claims

1. A sulfonium salt of the general formula (I)

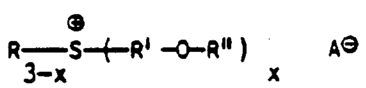

(I)

where
$A^{\ominus}$ is a non-nucleophilic counterion,
x is 1, 2 or 3,
R is alkyl, cycloalkyl, aryl, substituted aryl or, if x = 1, a divalent cyclic radical containing $S^{\oplus}$ as ring member,
R' is arylene or substituted arylene, and
R" is

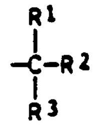

where $R^1$, $R^2$ and $R^3$ are identical or different and each is alkyl of from 1 to 18 carbon atoms or mono-halogenated or polyhalogenated alkyl, with the proviso that at least one of $R^1$ to $R^3$ contains a hydrogen atom on the α-carbon,

or
$R^1$ and $R^2$ are identical or different and each is hydrogen or alkyl, and $R^3$ is phenyl or substituted phenyl,
or
$R^1$ and $R^2$ are identical or different and each is hydrogen or alkyl, and $R^3$ is alkenyl or cycloalkenyl,
or
$R^1$ is hydrogen, and $R^2$ and $R^3$ form a preferably five- or six-membered ethylenically unsaturated ring,
or
$R^1$ and $R^2$ are identical or different and each is hydrogen, alkyl, cycloalkyl or aryl, and $R^3$ is alkoxy,

or $R^3$ combines with $R^2$ via the group $-O-(CH_2)_{\bar{n}}$ where n is from 3 to 6 to form a ring,
or
$R^1$ is hydrogen or alkyl and $R^2$ and $R^3$ are identical or different and each is alkoxy, aryloxy or substituted aryloxy.

2. A sulfonium salt as claimed in claim 1, wherein in the general formula-(I) R" is tert-alkyl or benzyl.

3. A sulfonium salt as claimed in claim 1, wherein in the general formula (I) R" is an allyl, ketal or acetal radical.

4. A radiation-sensitive mixture containing a sulfonium salt as claimed in any of the preceding claims.

5. A process for photoinitiated cationic polymerization, which comprises using a sulfonium salt as claimed in any of claims 1 to 3 as photoinitiator.

6. A process for producing relief patterns or relief images, which comprises using at least one sulfonium salt as claimed in any of claims 1 to 3 as photoinitiator and solubility inhibitor.

## Revendications

1. Sels de sulfonium de formule générale (I)

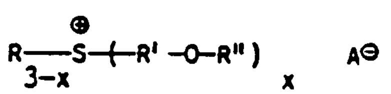

(I)

dans laquelle
$A^{\ominus}$ = ion compensateur non nucléophile,
x = 1, 2 ou 3,
R = alkyle, cycloalkyle, aryle, aryle substitué ou, lorsque x = 1, un restecyclique bivalent avec $S^{\oplus}$ comme chaînon cyclique,
R' = arylène, arylène substitué,
R" =

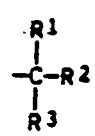

où $R^1$, $R^2$ et $R^3$ sont identiques ou différents et représentent alkyle à 1 à 18 atomes de carbone, alkyle substitué une ou plusieurs fois par halogène, étant entendu qu'au moins un des restes $R^1$ à $R^3$ contient, sur l'α-carbone, un atome d'hydrogène,
ou
$R^1$ et $R^2$ sont identiques ou différents et représentent hydrogène ou alkyle et $R^3$ est mis pour phényle ou phényle substitué,
ou

$R^1$ et $R^2$ sont identiques ou différents et représentent hydrogène ou alkyle et $R^3$ est mis pour alcényle ou cycloalcényle,
ou
$R^1$ est mis pour hydrogène et $R^2$ et $R^3$ forment un cycle à insaturation éthylénique, de préférence à cinq ou six chaînons,
ou
$R^1$ et $R^2$ sont identiques ou différents et représentent hydrogène, alkyle, cycloalkyle ou aryle et $R^3$ est mis pour alcoxy, ou $R^3$ avec $R^2$ forment un cycle par l'intermédiaire du groupe $-O-(CH_2)_n-$, n étant égal à 3 à 6,
ou
$R^1$ représente hydrogène ou alkyle, et $R^2$ et $R^3$ sont identiques ou différents et sont mis pour alcoxy, aryloxy ou aryloxy substitué.

2. Sels de sulfonium selon la revendication 1, caractérisés par le fait que dans la formule générale (I), R" est mis pour un reste ter-alkyle ou benzyle.

3. Sels de sulfonium selon la revendication 1, caractérisés par le fait que dans la formule générale (I), R" est mis pour un reste allyle, cétal ou acétal.

4. Mélange sensible au rayonnement, caractérisé par le fait qu'il contient un sel de sulfonium selon l'une des revendications précédentes.

5. Procédé de polymérisation cationique photo-sensibilisée, caractérisé par le fait que l'on utilise, comme photo-sensibilisateur, un sel de sulfonium selon l'une des revendications 1 à 3.

6. Procédé de préparation de modèles ou images en relief, caractérisé par le fait que l'on utilise , comme photo-sensibilisateur et inhibiteur de solubilité, au moins un sel de sulfonium selon l'une des revendications 1 à 3.